(19) Europäisches Patentamt
European Patent Office
Office européen des brevets

(11) **EP 1 563 294 B1**

(12) **EUROPÄISCHE PATENTSCHRIFT**

(45) Veröffentlichungstag und Bekanntmachung des Hinweises auf die Patenterteilung:
**13.12.2006 Patentblatt 2006/50**

(51) Int Cl.:
*G01N 33/36* (2006.01)　　*G01N 21/89* (2006.01)
*G01N 21/25* (2006.01)

(21) Anmeldenummer: 03757628.7

(22) Anmeldetag: **06.11.2003**

(86) Internationale Anmeldenummer:
**PCT/CH2003/000727**

(87) Internationale Veröffentlichungsnummer:
**WO 2004/044579 (27.05.2004 Gazette 2004/22)**

(54) **VORRICHTUNG ZUM ABTASTEN EINES GARNS MIT EINEM LICHTSTRAHL**

DEVICE FOR SCANNING A THREAD BY AN OPTICAL BEAM

DISPOSITIF DE BALAYAGE D'UN FIL AU MOYEN D'UN RAYON LUMINEUX

(84) Benannte Vertragsstaaten:
**BE CH DE ES FR IT LI**

(30) Priorität: **13.11.2002 CH 190102**

(43) Veröffentlichungstag der Anmeldung:
**17.08.2005 Patentblatt 2005/33**

(73) Patentinhaber: **Uster Technologies AG**
**8610 Uster (CH)**

(72) Erfinder: **OTT, Philipp**
**CH-8342 Wernetshausen (CH)**

(56) Entgegenhaltungen:
EP-A- 0 399 945　　　　EP-A- 0 761 585
EP-A- 1 018 645　　　　DE-A- 19 955 292

EP 1 563 294 B1

**Beschreibung**

Die Erfindung betrifft eine Vorrichtung zum Abtasten eines in seiner Längsrichtung in einem Messspalt bewegten Garns mit einem Lichtstrahl aus einer Lichtquelle, mit einem Empfänger für Licht, das am Garn reflektiert wird und mit einer Einheit zur Verarbeitung von elektrischen Signalen aus dem Empfänger.

Eine solche Vorrichtung ist beispielsweise aus der EP 0 761 585 bekannt, bei der eine Lichtquelle vorgesehen ist, die Licht abstrahlt, das am Garn einerseits reflektiert und andererseits auch abgeschattet wird. Das dabei von Empfängern empfangene Licht wird in an sich bekannter Weise in elektrische Signale gewandelt, für die Bereiche oder Schwellwerte vorgesehen werden können, um beispielsweise Fremdstoffe im Garn zu erkennen.
Ein Nachteil dieser Vorrichtung ist darin zu sehen, dass damit beispielsweise solche Fremdstoffe im Garn, welche die gleiche Farbe wie das ausgestrahlte Licht haben, nicht erkannt werden können. Dies trifft aber z.B. auch für durchsichtige Fremdstoffe wie Plastikfolienteile im Garn zu, so dass solche Fremdstoffe damit nicht erkannt werden können.

Eine weitere Vorrichtung dieser Art ist aus der WO 95/29396 bekannt, bei der einem Empfänger für weisses Licht drei verschiedene Lichtquellen zugeordnet sind. Diese drei Lichtquellen sind als drei lichtemittierende Dioden ausgebildet, wobei jede Diode Licht einer anderen Hauptwellenlänge im sichtbaren Bereich aussendet. Diese Lichtquellen sind in Längsrichtung des Garns gesehen hintereinander neben dem Garn angeordnet und so gerichtet, dass sie das Garn und einen dahinter liegenden, möglichst viel Licht absorbierenden Hintergrund beleuchten. Die durch Reflexion des Lichts am Garn im Empfänger entstehenden Signale werden so verarbeitet, dass aus den Werten, die für die Signale in den einzelnen Hauptwellenlängen ermittelt werden, Verhältniswerte gebildet werden, die wiederum an Kriterien gemessen werden können.
Ein Nachteil dieser Vorrichtung ist insbesondere darin zu sehen, dass sie nur für die Auswertung von reflektiertem Licht mit Garn vor einem vorzugsweise schwarzen Hintergrund taugt. Zudem benötigt die Anordnung von drei oder mehr Dioden in Abständen längs des Garns viel Raum, der möglicherweise an solchen Stellen in Textilmaschinen, die für solche Vorrichtungen vorgesehen sind, gar nicht vorhanden ist. Bei dieser Vorrichtung stellt sich zusätzlich das Problem, dass die Ansteuerung der einzelnen Lichtquellen auf die Bewegung des Garns Rücksicht nehmen und so vorgesehen sein muss, dass immer dieselbe Stelle am Garn mit Licht verschiedener Farbe beleuchtet wird. In EP1018645 werden hingegen eine Weißlichtquelle und drei farbempfindliche Sensoren verwendet, aus deren signalen Differenzen gebildet werden. Die Erfindung, wie sie in den Patentansprüchen gekennzeichnet ist, löst die Aufgabe, eine Vorrichtung der genannten Art zu schaffen, die es erlaubt, im Garn vorhandene Fremdstoffe selektiver und mit grösserer Empfindlichkeit zu erkennen.

Dies wird erfindungsgemäss mit einer Vorrichtung erreicht, die zur Abstrahlung von Licht in drei Wellenlängenbereichen eine einzige Lichtquelle vorsieht, wobei die Wellenlängenbereiche durch Hauptwellenlängen bestimmt sind. Die Hauptwellenlängen bestimmen drei Farben im Bereich von Wellenlängen des sichtbaren Lichts. Vorzugsweise sind dies die Farben Rot, Grün und Blau. Die Lichtquelle ist vorzugsweise als licht-emittierende Diode ausgebildet, die sichtbares Licht in drei Farben im sichtbaren Bereich getrennt abstrahlen kann. Die Lichtquelle und der Empfänger weisen Hauptachsen für die Abstrahlung und den Empfang von Licht auf, die zusammen eine Ebene aufspannen, die quer zur Längsrichtung des Garns steht. Die Einheit zur Verarbeitung von elektrischen Signalen aus dem Empfänger für reflektiertes Licht bildet aus den Signalen in jedem der drei vorgegebenen Wellenlängenbereichen einen Vektor in einem Raum und aus den Vektoren für die verschiedenen Signale einen Summenvektor. Für den Endpunkt des Summenvektors im Raum wird ein Bereich vorgegeben, der angibt, ob das zum Summenvektor verarbeitete elektrische Signal aus dem Empfänger einen Fremdstoff im Garn anzeigt. Der Raum, in dem die Vektoren berechnet und/oder dargestellt werden, bildet einen Würfel mit Achsen, längs denen Werte für die Intensität von drei Hauptwellenlängen aufgetragen sind.

Die Verwendung mehrerer identischer Empfänger für weisses Licht ermöglicht die Erfassung der Farben vom Garn ohne spektrale Fehler. Die Auswertung der erhaltenen Signale, die zum Ziele hat, einen Summenvektor aus den Anteilen der einzelnen Farben zu bilden, erlaubt es selektiv Fremdstoffe bestimmter Farben und Farbtöne zu erkennen, welche bestimmten Materialien entsprechen. Damit kann man aber auch Fremdstoffe gezielt im Garn belassen, indem man sie zuerst erkennt, oder indem man für den Endpunkt des Summenvektors einen Bereich so vorgibt, dass gewisse Fremdstoffe gar nicht erfasst werden. Durch die gezielte Reinigung des Garns im Hinblick auf bestimmte Verunreinigungen oder Fremdstoffe, kann die Leistung der Produktionsmaschinen wesentlich verbessert werden.

Im folgenden wird die Erfindung anhand eines Beispiels und mit Bezug auf die beiliegenden Figuren näher erläutert. Es zeigen:

Fig. 1 einen Schnitt durch eine erfindungsgemässe Vorrichtung,
Fig. 2, 3 und 4 je eine schematische Darstellung eines Teils der Vorrichtung in verschiedenen Phasen,
Fig. 5 eine schematische Darstellung von Wellenlängenbereichen,
Fig. 6 und 7 je eine schematische Darstellung der Auswertung der gemessenen Signale,
Fig. 8 bis 10 je eine Darstellung eines Zielbereiches für die Auswertung, und
Fig. 11 und 12 je Darstellungen von Möglichkeiten zur Ansteuerung der Lichtquelle der Vorrichtung.

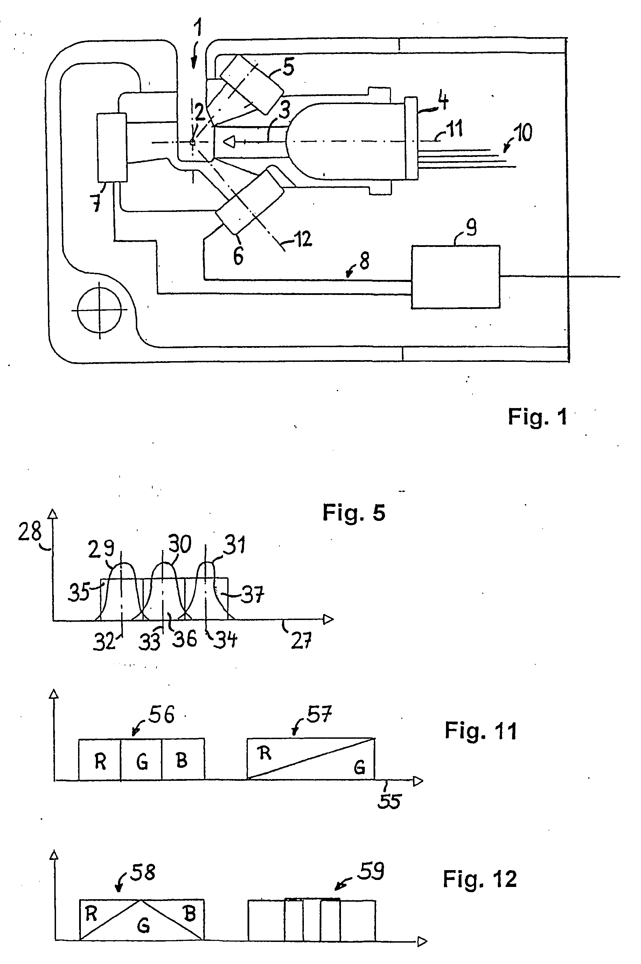

Fig. 1 zeigt eine erfindungsgemässe Vorrichtung wie z.B. einen Messkopf für die Messung von Garneigenschaften oder für einen Garnreiniger, mit einem Messspalt 1, in dem ein Garn 2 in seiner Längsrichtung bewegt wird, wobei diese Längsrichtung hier etwa senkrecht zur Zeichnungsebene gerichtet ist. Ein Lichtstrahl 3 wird von einer Lichtquelle 4 erzeugt und ist auf das Garn 2 gerichtet. Empfänger 5 und 6 sind für Licht angeordnet, das von der Oberfläche des Garns reflektiert wird. Als Lichtquelle ist eine lichtemittierende Diode, beispielsweise eine sogenannte RGB-LED vorgesehen, wie sie von der Firma Nichia (im Internet unter www.nichia.co.jp einsehbar) vom Typ NTSM 515 hergestellt wird. Es ist aber auch denkbar, andere Lichtquellen, z.B. auf Basis eines Lasers einzusetzen. Ein weiterer Empfänger 7 kann zusätzlich für Licht vorgesehen sein, das vom Garn 2 abgeschattet wird. Der oder die Empfänger 5, 6, 7 sind über je eine Leitung oder einen Bus 8 mit einer Einheit 9 zur Verarbeitung von elektrischen Signalen aus dem oder den Empfängern verbunden. Die Einheit 9 besteht aus einem Rechner mit einem Speicher, so z.B aus einem Mikroprozessor bekannter Bauart. Die Lichtquelle 4 weist vier Anschlüsse 10 auf, über die die einzelnen Wellenlängenbereiche oder Farben einzeln angesteuert werden können. In diesem Beispiel weisen die einzige Lichtquelle 4 und der Empfänger 6 Hauptachsen 11 und 12 für die Abstrahlung und den Empfang von Licht auf, die zusammen eine Ebene aufspannen, die quer zur Längsrichtung des Garns steht und hier der Zeichnungsebene entspricht.

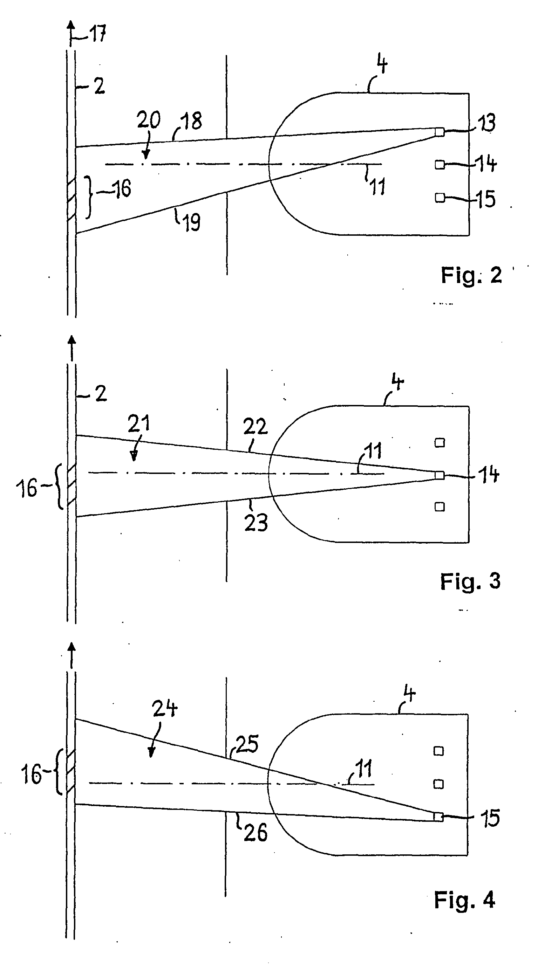

Fig. 2 zeigt in vereinfachter Darstellung die Lichtquelle 4 mit drei lichtemittierenden Dioden 13, 14 und 15 sowie das Garn 2 mit einem darin eingelagerten Fremdstoff 16. Bezogen auf die Hauptachse 11 der Lichtquelle 4 befindet sich der Fremdstoff kurz vor dieser Hauptachse, wenn man von einer Bewegungsrichtung ausgeht, wie sie ein Pfeil 17 angibt. Hier wird das Garn 2 mit dem Fremdstoff 16 beispielsweise durch rotes Licht von der Diode 13 belichtet, welche Licht in einem Bereiche 20 abstrahlt, wie er durch Linien 18 und 19 begrenzt wird.

Fig. 3 zeigt eine Darstellung gemäss der Fig. 2, wobei aber das Garn 2 mit dem Fremdstoff 16 sich etwa auf der Hauptachse 11 befindet. Hier wird das Garn 2 mit dem Fremdstoff 16 beispielsweise durch grünes Licht von der Diode 14 belichtet, welche Licht in einem Bereiche 21 abstrahlt, wie er durch Linien 22 und 23 begrenzt wird.

Fig. 4 zeigt eine Darstellung gemäss der Fig. 2, wobei aber das Garn 2 mit dem Fremdstoff 16 sich eher oberhalb der Hauptachse 11 befindet. Hier wird das Garn 2 mit dem Fremdstoff 16 beispielsweise durch blaues Licht von der Diode 15 belichtet, welche Licht in einem Bereiche 24 abstrahlt, wie er durch Linien 25 und 26 begrenzt wird.

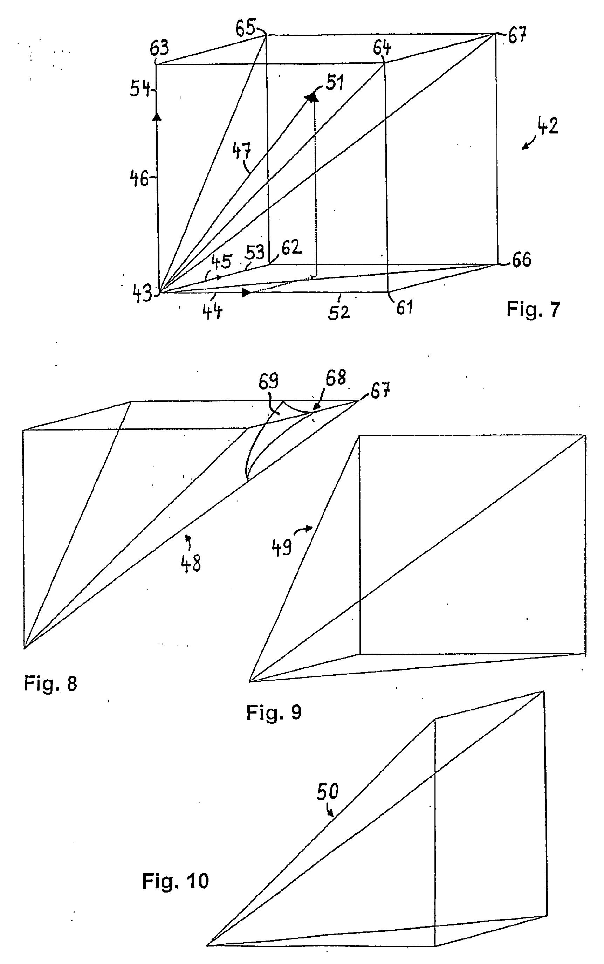

Fig. 5 zeigt eine Darstellung verschiedener Wellenlängenbereiche über einer Achse 27, längs der Werte für Wellenlängen aufgetragen werden können. Längs einer Achse 28 können Werte für die Intensität eines Signales in Funktion der Wellenlänge aufgetragen werden. Beispielsweise sind hier drei Wellenlängenbereiche 29, 30 und 31 durch Kurven aufgezeichnet, die den Verlauf der Intensität des ausgesendeten Lichts im Bereiche ihrer drei Hauptwellenlängen 32, 33 und 34 angeben. Vereinfacht und für nachfolgende Darstellungen, können diese Wellenlängenbereiche auch durch einfache Rechtecke 35, 36 und 37 dargestellt werden.

Fig. 6 zeigt eine Ebene 35, die durch Achsen 36 und 37 aufgespannt wird. Längs diesen Achsen 36, 37 können Werte für die Intensität je einer Farbe des vom Garn reflektierten Lichts der Lichtquelle 4 als Vektoren aufgetragen werden. Daraus kann ein Summenvektor 38 berechnet werden. In der Ebene 35 können Bereiche 39, 40, 41 vorgegeben sein, die für bestimmte Eigenschaften des Garns stehen. Solche Eigenschaften können die Art der Grundmaterialien aus denen das Garn besteht oder die Art der Fremdstoffe sein, die im Garn vorkommen.

Analog zu Fig. 6 welche für Licht aus zwei verschiedenen Wellenlängenbereichen gilt, zeigt Fig. 7 einen als Würfel abgegrenzten Raum 42 für die Darstellung von Vektoren, die der Intensität der empfangenen Signale in drei verschiedenen Wellenlängenbereichen entsprechen. Eine Ecke 43 des Raumes soll hier als Ausgangspunkt für Vektoren 44, 45, 46 dienen, von denen jeder für einen bestimmten Wellenlängenbereich steht. Mit 47 ist ein Summenvektor bezeichnet, der aus den drei Vektoren 44, 45 und 46 zusammengesetzt ist. Dieser Raum oder Würfel 42 kann in verschiedene Bereiche unterteilt werden. Je ein solcher Bereich 48 ist in Fig. 8, ein Bereich 49 in Fig. 9 und ein Bereich 50 in Fig.10 dargestellt. Diese Bereiche 48, 49 und 50 sind als Zielgebiet für den Endpunkt 51 des Summenvektors 47 gedacht. Je nach dem ob dieser Endpunkt 51 in einem dieser Bereiche 48, 49, 50 liegt oder nicht, gilt, dass eine bestimmte Bedingung für eine Eigenschaft des Garns, wie beispielsweise die Anwesenheit eines bestimmten Fremdstoffes erfüllt ist oder nicht. Der Raum 42, der hier als Würfel abgebildet ist, wird durch Achsen 52, 53 und 54 gebildet, längs denen Werte für die Intensität von drei Hauptwellenlängen aufgetragen sind.

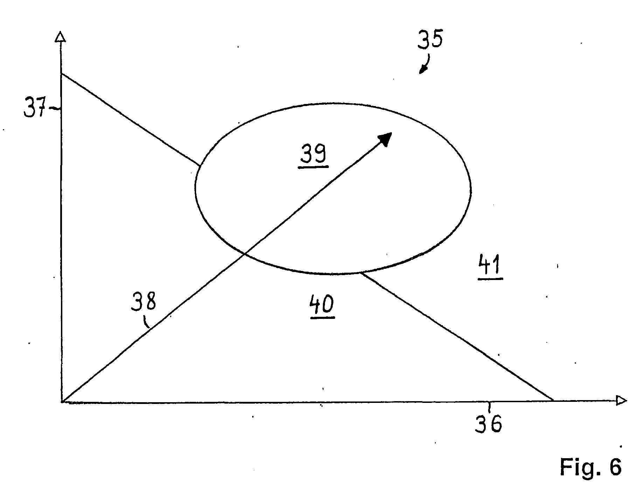

Fig. 11 zeigt verschiedene Wellenlängenbereiche, wie sie aus der Fig. 5 bekannt sind, nun über einer Zeitachse 55 aufgetragen. Das ergibt Beispiele für mögliche, sich wiederholende Sequenzen für die Abstrahlung von Licht durch die Lichtquelle 4. Gemäss einer Sequenz 56, soll die Lichtquelle 4 nacheinander Licht mit den Farben Rot, Grün und Blau abstrahlen. Eine Sequenz 57 gibt an, dass über eine vorgegebene Zeit Licht roter Farbe mit abnehmender Intensität und Licht grüner Farbe mit zunehmender Intensität gleichzeitig auszustrahlen ist. Arbeitet man mit drei Farben, so ergeben sich so zwei oder drei Sequenzen mit je zwei Farben. Nach Ablauf der drei Sequenzen beginnt man wieder mit der ersten Sequenz 57.

Fig. 12 zeigt weitere Sequenzen analog zu Fig. 11. Eine Sequenz 58 für drei Farben die sich überlappen, die aber zusammen jeweils eine vorgegebene Gesamtintensität ergeben. Eine Sequenz 59 betrifft wieder drei Farben die mit R, G und B bezeichnet sind und sich überlappen. Dabei ist die Intensität der Signale in den drei Farben immer konstant oder maximal.

Die Wirkungweise der Vorrichung soll nachfolgend ergänzend beschreiben werden, soweit sie nicht bereits aus den obigen Beschreibungsteilen erkennbar ist. Um beispielsweise einen bestimmten Fremdstoff oder eine andere Eigenschaft im oder am Garn 2 zu erkennen, wird die Lichtquelle über die Anschlüsse 10 beispielsweise so angesteuert, dass sie Licht in nur einem begrenzten Wellenlängenbereich abgibt. Dieses wird am Garn reflektiert und auf einen Empfänger 5, 6 zurückgeworfen, der dieses empfängt und in ein elektrisches Signal umwandelt, das er über die Leitung oder den Bus 8 an die Einheit 9 übermittelt, die das Signal oder einen daraus abgeleiteten numerischen Wert speichert. Unmittelbar darauffolgend wird die Lichtquelle 4 so angesteuert, dass sie Licht in einem weiteren Wellenlängenbereich abstrahlt u.s.w., so dass schliesslich in der Einheit 9 ein weiteres Signal oder ein weiterer Wert gespeichert werden kann. Aus den gespeicherten Werten werden im Rechner der Einheit 9 Vektoren 44, 45 und eventuell 46 und daraus schliesslich ein Summenvektor 38 oder 47 gebildet. In der Einheit 9 sind Werte gespeichert, die in der Ebene 35 oder im Raum 42 mindestens einen Bereich 39, 40, 41 oder 48, 49, 50 definieren, so dass es durch einen Vergleich möglich ist, festzustellen ob der Endpunkt des Summenvektors in einem dieser Bereiche liegt oder nicht. Als Resultat eines solchen Vergleiches kann über eine Leitung 60 dann von der Einheit 9 ein Signal ausgegeben werden, das angibt, ob beispielsweise eine gesuchte Eigenschaft vorliegt, beispielsweise ob ein Fremdstoff im Garn vorhanden ist oder nicht.

Ist ein weiterer Empfänger 7 für Durchlicht vorhanden, so kann dieser beispielsweise ein weiteres Signal an die Einheit 9 abgeben, das eine Angabe über den Durchmesser des Garns macht. So lässt sich in bekannter Weise auch der Einfluss des Durchmessers des Garns auf das empfangene reflektierte Licht ausgleichen.

Wie bereits in den Figuren 11 und 12 gezeigt, gibt es verschiedene Möglichkeiten die Lichtquelle 4 anzusteuern. Verwendet man beispielsweise laufende Übergänge zwischen zwei oder mehr Wellenlängenbereichen, wie dies die Sequenzen 57 und 58 zeigen, so kann man auch zu verschiedenen Zeiten während der Sequenz die Vektoren und den Summenvektor 38, 47 bestimmen. So vollführt der Summenvektor 38, 47 in der Ebene oder im Raum während der Sequenz eine Bewegung und es ist möglich, dabei die Zeiten oder die Anteile der Wellenlängenbereiche zu erfassen, bei denen der Endpunkt in einem bestimmten Bereiche 22 liegt. So kann man auch ausgehend von gemischtem Licht aus zwei Wellenlängenbereichen Erkenntnisse über Eigenschaften des Garns gewinnen, die sonst nicht erhältlich sind.

Für eine getrennte Beleuchtung des Garns gemäss einer Sequenz 56, soll die Taktfrequenz, mit der die einzelnen Dioden 13, 14, 15 der Lichtquelle 4 anzusteuern sind, mindestens so gross sein, dass eine bestimmte Stelle, wie beispielsweise der Ort an dem ein Fremdstoff 16 im Garn 2 eingelagert ist, sich in einer bestimmten Zeit nur soweit bewegt, dass er noch in die Bereiche 20, 21 und 24 fällt. Diese bestimmte Zeit dauert hier drei Takte oder ein Mehrfaches davon. Durch die extrem nahe räumliche Anordnung der Dioden 13 bis 15 in der einzigen Lichtquelle 4 ist die für die Beleuchtung mit drei Wellenlängenbereichen benötigte Zeit sehr kurz und somit ist es leicht möglich eine solche Stelle dreimal zu erfassen, während der sie sich vor der Lichtquelle 4 befindet.

Wenn als Wellenlängenbereiche die Farben Rot, Grün und Blau gewählt werden, so kann man in der Darstellung gemäss Fig. 7 diese und weitere Farben im Raum 42 folgendermassen angeordnet erkennen:

In der Ecke 43 soll die Intensität der schwarzen Farbe maximal sein. In der Ecke 61 soll die Intensität der roten Farbe maximal sein. In der Ecke 62 soll die Intensität der grünen Farbe maximal sein. In der Ecke 63 soll die Intensität der blauen Farbe maximal sein. In der Ecke 64 soll die Intensität der Farbe Magenta maximal sein. In der Ecke 65 soll die Intensität der Farbe Cyan maximal sein. In der Ecke 66 soll die Intensität der Farbe Gelb maximal sein und in der Ecke 67 soll die Intensität der weissen Farbe maximal sein.

Es ist an sich bekannt aus welchen Formeln die genannten Vektoren berechnet werden können. Der Vollständigkeit halber sei hier noch ein Beispiel angefügt. Für die Länge des Summenvektors 47, wie er aus der Fig. 7 bekannt ist und den wir hier mit $V_{47}$ bezeichnen, gilt:

$$|V_{47}| = \sqrt{|V_{44}|^2 + |V_{45}|^2 + |V_{46}|^2}$$

wobei mit $V_{44}, V_{45}$ und $V_{46}$ die Längen der Vektoren 44, 45 und 46 benannt sind. Die Richtung des Summenvektors bestimmt sich nach bekannten Regeln der Trigonometrie. Eine entsprechend angepasste Berechnung kann für die Vektoren der Fig. 6 angewendet werden.

Aus der Lage des Summenvektors 38, 47 kann nun beispielsweise ein Hinweis darauf gewonnen werden, ob ein Fremdstoff vorhanden ist, welcher Art er ist und ob er stört und beseitigt werden muss oder nicht. Beispielsweise kann gemäss Fig. 6 bestimmt werden, dass der Summenvektor 38 in Fig. 6 für tolerierte Fremdstoffe nur mit seinem Endpunkt im Bereiche 41 oder sogar im Bereiche 39 liegen soll. In den Figuren 7 - 10 könnte man beispielsweise annehmen, dass der Endpunkt 51 des Summenvektors 47 in einem Bereiche 68, der durch eine Fläche 69 abgegrenzt wird und nahe der Ecke 67 liegen sollte, wenn kein Fremdstoff vorhanden ist und das Garn weiss oder nahezu weiss ist. Liegt dieser Endpunkt 51 in den Bereichen 48, 49 oder 50, so geht man davon aus, dass der Fremdstoff vorwiegend blau, grün oder rot ist. Dabei kann es aber trotzdem sein, dass ein solcher Fremdstoff an sich tolerierbar ist. Das kann beispielsweise für einen roten Fremdstoff dann der Fall sein, wenn das Garn anschliessend rot gefärbt werden soll.

**Patentansprüche**

1. Vorrichtung zum Abtasten eines in seiner Längsrichtung in einem Messspalt (1) bewegten Garns (2) mit einem Lichtstrahl (3) aus einer Lichtquelle, mit einem Empfänger (5, 6) für Licht, das am Garn reflektiert wird und mit einer Einheit (9) zur Verarbeitung von elektrischen Signalen aus dem Empfänger, wobei zur Abstrahlung von Licht in drei Wellenlängenbereichen (29, 30, 31) eine einzige Lichtquelle (4) vorgesehen ist, wobei die Wellenlängenbereiche durch drei Hauptwellenlängen (32, 33, 34) bestimmt sind und die Einheit (9) zur Verarbeitung von elektrischen Signalen aus dem Empfänger für empfangenes Licht einen Rechner aufweist, der aus den Werten für jeden der drei vorgegebenen Wellenlängenbereiche einen Vektor (44, 45, 46) und aus den Vektoren einen Summenvektor (47) bildet, **dadurch gekennzeichnet, dass** für den Endpunkt (51) des Summenvektors (47) in einem Raum (42) ein Bereich (48, 49, 50) abgegrenzt ist, der angibt, ob das zum Summenvektor verarbeitete elektrische Signal aus dem Empfänger einen Fremdstoff im Garn anzeigt und der Raum einen Würfel bildet, der durch Achsen (52, 53, 54) gebildet ist, längs denen Werte für die Intensität von drei Hauptwellenlängen aufgetragen sind.

2. Vorrichtung nach Anspruch 1, **dadurch gekennzeichnet, dass** die Hauptwellenlängen Farben im Bereich von Wellenlängen des sichtbaren Lichtes bestimmen.

3. Vorrichtung nach Anspruch 2, **dadurch gekennzeichnet, dass** die Hauptwellenlängen die Farben Rot, Grün und Blau betreffen.

4. Vorrichtung nach Anspruch 1, **dadurch gekennzeichnet, dass** die einzige Lichtquelle (4) als lichtemittierende Diode ausgebildet ist, die sichtbares Licht in drei Farben im sichtbaren Bereich getrennt abstrahlen kann.

5. Vorrichtung nach Anspruch 1, **dadurch gekennzeichnet, dass** die einzige Lichtquelle und ein Empfänger (6) Hauptachsen (11, 12) für die Abstrahlung und den Empfang von Licht aufweisen, die zusammen eine Ebene aufspannen, die quer zur Längsrichtung des Garns steht.

**Claims**

1. Device for scanning yarn (2), which is moved in its longitudinal direction in a measurement gap (1), with a light beam (3) from a light source, comprising a receiver (5, 6) for light that is reflected on the yarn and comprising a unit (9) for processing electrical signals from the receiver, whereas a single light source (4) is provided for emitting light in three wavelength ranges (29, 30, 31), the wavelength ranges being determined by three principal wavelengths (32, 33, 34) and the unit (9) for processing electrical signals from the receiver for received light comprises a computer, which forms a vector (44, 45, 46) from the values for each of the three predetermined wavelength ranges and a sum vector (47) from the vectors, **characterised in that** a region (48, 49, 50) is delimited for the end point (51) of the sum vector (47) in space (42) that indicates whether the electrical signal, which is processed to form a sum vector from the receiver displays a foreign substance in the yarn and **in that** said space forms a cube, which is formed by axes (52, 53, 54) along which values for the intensity of three principal wavelengths are plotted.

2. Device according to claim 1, **characterised in that** the principal wavelengths determine colours in the range of wavelengths of visible light.

3. Device according to claim 2, **characterised in that** the principal wavelengths relate to the colours red, green and blue.

4. Device according to claim 1, **characterised in that** the single light source (4) is configured as a light-emitting diode, which can separately emit visible light in three colours of the visible range.

5. Device according to claim 1, **characterised in that** the single light source (4) and a receiver (6) exhibit principal axes (11, 12) for emitting and receiving light that together enclose a plane, which is located transversely to the longitudinal direction of the yarn.

**Revendications**

1. Dispositif de balayage d'un fil (2) déplacé dans sa direction longitudinale dans une fente de mesure (1) par un rayon lumineux (3) provenant d'une source de lumière, avec un récepteur (5, 6) pour la lumière qui est reflétée sur le fil et avec une unité (9) pour le traitement des signaux électriques provenant du récepteur, dans lequel, pour l'émission de la lumière dans trois intervalles de longueur d'onde (29, 30, 31), une seule source de lumière (4) est prévue, sachant que les intervalles de longueur d'onde sont déterminés par trois longueurs d'onde principales (32, 33, 34) et que l'unité (9) pour le traitement des signaux électriques provenant du récepteur pour la lumière reçue comporte un calculateur qui forme un vecteur (44, 45, 46) à partir des valeurs pour chacun des trois intervalles de longueur d'onde prédéfinis, et à partir des vecteurs un vecteur somme (47), **caractérisé en ce que**, pour le point d'extrémité (51) du vecteur somme (47), un domaine (48, 49, 50) est limité dans un espace (42), domaine qui spécifie si le signal du récepteur traité en un vecteur somme signale un corps étranger dans le fil et **en ce que** l'espace forme un cube qui est formé par les axes (52, 53, 54) le long desquels sont inscrites des valeurs pour l'intensité de trois longueurs d'onde principales.

2. Dispositif selon la revendication 1, **caractérisé en ce que** les longueurs d'onde principales déterminent des couleurs dans le domaine de longueurs d'onde de la lumière visible.

3. Dispositif selon la revendication 2, **caractérisé en ce que** les longueurs d'onde principales concernent les couleurs rouge, verte et bleue.

4. Dispositif selon la revendication 1, **caractérisé en ce que** la seule source de lumière (4) est configurée comme diode électroluminescente qui peut rayonner de la lumière visible séparément en trois couleurs dans le domaine visible.

5. Dispositif selon la revendication 1, **caractérisé en ce que** la seule source de lumière et un récepteur (6) comportent pour l'émission et la réception de la lumière des axes principaux (11, 12) qui sous-tendent ensemble un plan qui est disposé transversalement par rapport à la direction longitudinale du fil.

Fig. 1

Fig. 5

Fig. 11

Fig. 12

Fig. 2

Fig. 3

Fig. 4

Fig. 7

Fig. 8

Fig. 9

Fig. 10

9

Fig. 6